(19)

Europäisches
Patentamt
European
Patent Office
Office européen
des brevets

(11) **EP 4 706 696 A1**

(12) **EUROPEAN PATENT APPLICATION**
published in accordance with Art. 153(4) EPC

(43) Date of publication:
**11.03.2026  Bulletin 2026/11**

(21) Application number: **24799900.6**

(22) Date of filing: **30.04.2024**

(51) International Patent Classification (IPC):
**A61L 27/36** *(2006.01)*    **A61L 27/50** *(2006.01)*
**A61K 35/35** *(2015.01)*

(52) Cooperative Patent Classification (CPC):
**A61K 35/35; A61L 27/36; A61L 27/50; A61L 27/56**

(86) International application number:
**PCT/CN2024/090990**

(87) International publication number:
**WO 2024/227443 (07.11.2024 Gazette 2024/45)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **04.05.2023  CN 202310490009**

(71) Applicant: **Shanghai Sondray Biotechnology Co.,
Ltd.
Shanghai 200241 (CN)**

(72) Inventors:
• YANG, Chengran
  Shanghai 200241 (CN)
• ZHANG, Wenjie
  Shanghai 200241 (CN)

(74) Representative: **Mewburn Ellis LLP
Aurora Building
Counterslip
Bristol BS1 6BX (GB)**

(54) **DECELLULARIZED ADIPOSE BIOMATERIAL AND METHOD FOR PREPARING SAME**

(57)    The present invention provides a decellularized adipose biomaterial. Specifically, the decellularized adipose biomaterial is a cell-free extract derived from an adipose tissue and a biomaterial acquired by mixing a liquid cell-free fat extract of a fat mixture acquired by washing, crushing and separating an adipose tissue with a solid cell-free fat extract acquired from a fat mixture by decellularization, delipidation and other processes. The decellularized adipose biomaterial prepared by mixing has long-acting tissue repair and tissue regeneration-guiding capability, and can be used in the fields of soft tissue defect repair, shaping and filling, and the like.

Figure   1

EP 4 706 696 A1

## Description

## FIELD OF THE INVENTION

The present invention relates to the field of biomedical materials, particularly to a decellularized adipose biomaterial and method for preparing same.

## BACKGROUND OF THE INVENTION

Autologous tissue transplantation is often considered the gold standard for tissue repair, but the limited amount of autologous material and the potential for secondary damage to patients make autologous tissue transplantation have certain limitations. The best scaffold for tissue repair is the extracellular matrix of the target tissue. So far, the commercialized allogeneic extracellular matrix products mainly include dermis, tendons, nerves, and bone tissue. Compared to heterologous materials, the acquisition of human tissue is very valuable and usually comes from body donation projects.

In liposuction surgery, a large amount of adipose tissue is often discarded as medical waste. Research has shown that decellularized adipose matrix extracted from adipose can be used for *in vitro* adipose tissue engineering and *in vivo* adipose tissue regeneration. It is not limited to adipose tissue engineering, and also has potential applications in wound healing, bone regeneration, supporting breast tissue reconstruction, and promoting nerve repair.

Although research in this field has made significant progress, there are still many issues that need to be addressed and further studied.

Commercial decellularized products have very high requirements for virus inactivation and immunogenicity clearance. However, the current complex process of removing cellular material and adipose, clearing viruses and other immunogenic substances from adipose tissue not only decellularizes, but also removes active protein factors, greatly reducing the inherent biological activity of decellularized adipose products, thereby affecting the ability of materials to guide tissue repair and regeneration.

Therefore, it is necessary to provide a new decellularized adipose biomaterial and method for preparing same to address the shortcomings of prior art.

## SUMMARY OF THE INVENTION

The object of the present invention is to provide a decellularized adipose biomaterial and method for preparing same, particularly to provide a biomaterial with tissue repair and tissue regeneration-guiding capability and method for preparing same, which can be used in the fields of soft tissue defect repair, plastic surgery and filling, and the like.

In the first aspect of the present invention, provided is a decellularized adipose biomaterial, the decellularized adipose biomaterial is prepared by a method comprising the steps of:

(1) providing an adipose tissue raw material, crushing the adipose tissue raw material, and rinsing it to obtain a rinsed adipose tissue;

(2) subjecting the rinsed adipose tissue to centrifugation to obtain a layered mixture, removing the upper oil layer and the lower aqueous layer, and collecting the intermediate layer;

(3) subjecting the intermediate layer to emulsification to obtain an emulsified fat mixture;

(4) subjecting the emulsified fat mixture to centrifugation to obtain an intermediate liquid layer, which is the initial adipose extract A, and an intermediate solid layer, which is the initial adipose extract B;

(5) subjecting the initial adipose extract A to filtration and sterilization to obtain cell-free fat extract C;

(6) subjecting the initial adipose extract B to emulsification treatment, disinfection treatment, decellularization treatment, and delipidation treatment in sequence to obtain cell-free fat extract D;

(7) mixing the cell-free fat extract C with D and post-processing to obtain the decellularized adipose biomaterial.

In another preferred embodiment, the post-processed decellularized adipose biomaterial is a solid material.

In another preferred embodiment, the post-processed decellularized adipose biomaterial is a block material.

In another preferred embodiment, the decellularized adipose biomaterial is a porous material.

In another preferred embodiment, the decellularized adipose biomaterial has a pore size of 1-1000 $\mu$m.

In another preferred embodiment, the decellularized adipose biomaterial is free of cells and free of lipid droplets.

In another preferred embodiment, the lipid droplets are oil droplets released from ruptured adipocytes.

In another preferred embodiment, "free of lipid droplets" means that in the decellularized adipose biomaterial, the volume of oil droplets accounts for less than 1% of the total liquid, preferably less than 0.5%, and more preferably less than 0.1%.

In another preferred embodiment, the cells are selected from the group consisting of endothelial cells, adipose derived stem cells, macrophages, and stromal cells.

In another preferred embodiment, "free of cells" means that the average number of cells in 1ml of decellularized adipose biomaterial is ≤ 1, preferably ≤ 0.5, more preferably ≤ 0.1, or 0.

In another preferred embodiment, in step (1), physiological saline is used for rinsing.

In another preferred embodiment, in step (2), the centrifugation is performed at 800-2500g, preferably 800-2000g, more preferably 1000-1500g, and most preferably 1100-1300g.

In another preferred embodiment, in step (2), the centrifugation time is 1-15 minutes, preferably 1-10 minutes, more preferably 1-8 minutes, and most preferably 1-5 minutes.

In another preferred embodiment, in step (2), the centrifugation is performed at 0-10 °C, preferably at 2-5 °C, such as 4 °C.

In another preferred embodiment, in step (2), the intermediate layer is an adipose layer containing adipocytes.

In another preferred embodiment, in step (2), the layered mixture is divided into three layers, with the upper layer being the oil layer, the intermediate layer being the adipose layer containing adipocytes, and the lower layer being the aqueous layer.

In another preferred embodiment, in step (3), the emulsification is mechanical emulsification.

In another preferred embodiment, the mechanical emulsification is carried out by repeatedly pipetting with a syringe (such as pipetting 20-200 times, preferably 20-150 times, more preferably 20-100 times, and more preferably 30-50 times).

In another preferred embodiment, the pipetting method is that two 10ml injection syringes are connected to a three-way stopcock and repeatedly push-pull mixed at a constant speed.

In another preferred embodiment, in step (3), the emulsification comprises breaking and emulsifying by a tissue homogenizer.

In another preferred embodiment, in step (3), the speed of the homogenizer is 5000-15000 r/min, preferably 8000-15000 r/min, such as 10000 r/min, 12000 r/min.

In another preferred embodiment, in step (3), the emulsification time is 0.5-15 minutes, preferably 1-10 minutes, more preferably 1-8 minutes, most preferably 1-5 minutes, such as 3 minutes.

In another preferred embodiment, step (4) further comprises freezing and thawing the emulsified fat mixture before centrifuging it.

In another preferred embodiment, after freezing and thawing treatment, the thawed mixture is subjected to centrifugation.

In another preferred embodiment, the freezing temperature is -50 °C to -120 °C, preferably -60 °C to -100 °C, and more preferably -70 °C to -90 °C.

In another preferred embodiment, the thawing temperature is 20-40 °C, preferably 25-40 °C, and more preferably 37 °C.

In another preferred embodiment, the thawing is carried out under water bath conditions of 20-40 °C (preferably 25-40 °C, more preferably 37 °C).

In another preferred embodiment, the number of freeze-thaw cycles is 1-5 times (preferably 1, 2, 3, or 4 times).

In another preferred embodiment, in step (4), after centrifugation, the emulsified fat mixture is layered into 4 layers, with the first layer being an oil layer, the second layer being a residual adipose tissue layer, the third layer being a liquid layer, and the fourth layer being a cell/tissue fragment precipitation layer.

In another preferred embodiment, the intermediate solid layer in step (4) is the second layer, which is the residual adipose tissue layer.

In another preferred embodiment, the intermediate liquid layer in step (4) is the third layer, i.e. the liquid layer.

In another preferred embodiment, in step (4), the centrifugation is carried out at 800-2500g, preferably 800-2000g, more preferably 1000-1500g, and most preferably 1100-1300g.

In another preferred embodiment, in step (4), the centrifugation time is 1-15 minutes, preferably 1-10 minutes, more preferably 2-8 minutes, and most preferably 3-7 minutes.

In another preferred embodiment, in step (4), the centrifugation is performed at 0-10 °C, preferably at 2-5 °C, such as 4 °C.

In another preferred embodiment, in step (4), the first layer, second layer, third layer, and fourth layer are sequentially arranged from top to bottom.

In another preferred embodiment, in step (4), the intermediate liquid layer is a transparent or substantially transparent layer.

In another preferred embodiment, in step (4), the intermediate solid layer is yellow or brown.

In another preferred embodiment, in step (5), the filtration removes adipocytes or other potentially mixed live cells from the initial adipose extract A.

In another preferred embodiment, in step (5), the filtration and sterilization are performed using a filter.

In another preferred embodiment, the filter is a microporous membrane filter.

In another preferred embodiment, the pore size of the microporous membrane filter is 0.05-0.8 $\mu$m, preferably 0.1-0.5 $\mu$m, more preferably 0.1-0.4 $\mu$m, more preferably 0.15-0.3 $\mu$m, more preferably 0.2-0.25 $\mu$m, and most preferably 0.22 $\mu$m.

In another preferred embodiment, in step (5), the filtration and sterilization includes first passing through a first filter capable of filtering out cells, and then passing through a second filter (such as a 0.22 $\mu$m filter) capable of filtering out pathogens (such as bacteria).

In another preferred embodiment, step (5) further comprises aliquoting the fat extract C to form an aliquoted mixture.

In another preferred embodiment, the aliquoted extract is stored frozen or refrigerated.

In another preferred embodiment, the aliquoted extract is frozen and stored at -20 °C for later use, preferably, the aliquoted extract is directly used after thawed at low temperature (such as -4 °C) or at room temperature after being frozen and stored, or used after stored at low temperature (such as 4 °C) for a period of time after thawing.

In another preferred embodiment, step (6) comprises:

(6a) subjecting the initial adipose extract B to emulsification treatment, and post-processing to obtain a solid content;
(6b) subjecting the solid content obtained in step (6a) to disinfection treatment, and post-processing to obtain a solid content;
(6c) subjecting the solid content obtained in step (6b) to decellularization treatment, and post-processing to obtain a solid content;
(6d) subjecting the solid content obtained in step (6c) to delipidation treatment, and post-processing to obtain a solid content;
(6e) diluting the solid content obtained in step (6d) to obtain cell-free fat extract D.

In another preferred embodiment, in step (6) or step (6a), the emulsification treatment comprises mechanical emulsification.

In another preferred embodiment, in step (6) or step (6a), the emulsification treatment comprises homogenizing and emulsifying with a homogenizer.

In another preferred embodiment, the speed of the homogenizer is 5000-15000 r/min, preferably 8000-15000 r/min, such as 10000 r/min, 12000 r/min.

In another preferred embodiment, in step (6) or step (6a), the emulsification time is 0.5-15 minutes, preferably 1-10 minutes, more preferably 1-8 minutes, and most preferably 1-5 minutes.

In another preferred embodiment, in step (6), the emulsification treatment further comprises a step of centrifuging to separate the oil layer.

In another preferred embodiment, the centrifugation is carried out at 1000-8000 r/min, preferably 2000-5000 r/min, and more preferably 4000 r/min.

In another preferred embodiment, the centrifugation time is 2-20 minutes, preferably 5-15 minutes, and more preferably 10 minutes.

In another preferred embodiment, step (6) further comprises a step of centrifuging to separate the oil layer to obtain a solid content after the emulsification treatment.

In another preferred embodiment, step (6a) comprises homogenizing and mechanically emulsifying the initial adipose extract B, separating the oil layer after centrifugation to obtain a solid content.

In another preferred embodiment, in step (6) or step (6b), the disinfection treatment comprises a step of mixing with a disinfectant and disinfecting.

In another preferred embodiment, the mixing comprises stirring, oscillating.

In another preferred embodiment, in step (6) or step (6b), the disinfection treatment comprises mixing with a disinfectant, oscillating and then discarding the liquid layer.

In another preferred embodiment, in step (6) or step (6b), the disinfection treatment comprises disinfecting under irradiation, oscillating and then discarding the liquid layer.

In another preferred embodiment, the disinfectant is a mixed solution of peracetic acid and ethanol, or supercritical carbon dioxide.

In another preferred embodiment, the concentration of peracetic acid is 0.05-3%, preferably 0.1-2%, more preferably 0.1-1%, such as 0.5%.

In another preferred embodiment, the concentration of ethanol is 1-24%, preferably 2-20%, more preferably 2-10%, such as 4%, 5%, or 8%.

In another preferred embodiment, the mass ratio of the solid content obtained after emulsification treatment to the disinfectant is 1:1~1:20, preferably 1:2~1:20, more preferably 1:5~1:20, such as 1:8, 1:10.

In another preferred embodiment, the oscillation treatment time is 0.5-6 hours, preferably 1-6 hours, such as 4 hours.

In another preferred embodiment, step (6) or step (6b) comprises a post-processing step of rinsing the obtained solid content after the disinfection treatment.

In another preferred embodiment, the rinsing comprises oscillating and washing with PBS solution, and/or oscillating and washing with purified water.

In another preferred embodiment, step (6b) comprises mixing the solid content obtained in step (6a) with a disinfectant, discarding the liquid layer, and rinsing to obtain a solid content.

In another preferred embodiment, in step (6) or step (6c), the decellularization treatment comprises a step of mixing with a decellularizing agent.

In another preferred embodiment, the mixing comprises stirring, or oscillating.

In another preferred embodiment, in step (6) or step (6c), the decellularization treatment comprises mixing with a decellularizing agent, oscillating and then discarding the liquid layer.

In another preferred embodiment, the decellularizing agent is selected from the group consisting of SDS, TritonX-100, sodium deoxycholate, and the combination thereof.

In another preferred embodiment, the concentration of the decellularizing agent is 0.2-5%, preferably 0.5-2%, more preferably 0.8-1.5%, such as 1%.

In another preferred embodiment, the mass ratio of the solid content obtained after disinfection treatment to the decellularizing agent is 1:1~1:20, preferably 1:2~1:20, more preferably 1:5~1:20, such 1:8, 1:10.

In another preferred embodiment, the oscillation treatment time is 12-96 hours, such as 12 hours, 24 hours, 48 hours, or 72 hours.

In another preferred embodiment, step (6) or step (6c) comprises a post- processing step of rinsing the obtained solid content after decellularization treatment.

In another preferred embodiment, the rinsing comprises oscillating and washing with PBS solution, and/or oscillating and washing with purified water.

In another preferred embodiment, step (6c) comprises mixing the solid content obtained in step (6b) with a decellularizing agent, discarding the liquid layer, and rinsing to obtain a solid content.

In another preferred embodiment, in step (6) or step (6d), the delipidation treatment comprises a step of mixing with a degreaser.

In another preferred embodiment, the mixing comprises stirring, or oscillating.

In another preferred embodiment, in step (6) or step (6d), the delipidation treatment comprises mixing with a degreaser, oscillating and then discarding the liquid layer.

In another preferred embodiment, the degreaser is selected from the group consisting of isopropanol, ethanol, ether, and combinations thereof.

In another preferred embodiment, the mass ratio of the solid content obtained after decellularization treatment to the degreaser is 1:1~1:20, preferably 1:2~1:20, more preferably 1:5~1:20, such as 1:6, 1:8, or 1:10.

In another preferred embodiment, the oscillation treatment time is 0.5-24 hours, preferably 2-16 hours, such as 3 hours, 4 hours, or 8 hours.

In another preferred embodiment, step (6) or step (6d) comprises a post-processing step of rinsing the obtained solid content after the delipidation treatment.

In another preferred embodiment, the rinsing comprises oscillating and washing with PBS solution, and/or oscillating and washing with purified water.

In another preferred embodiment, step (6d) comprises mixing the solid content obtained in step (6c) with a degreaser, discarding the liquid layer, and rinsing to obtain a solid content.

In another preferred embodiment, the emulsification treatment, disinfection treatment, decellularization treatment, and delipidation treatment optionally further include a post-processing treatment step of washing.

In another preferred embodiment, the washing comprises sequentially washing with PBS solution, and purified water.

In another preferred embodiment, step (6e) comprises mixing the solid content obtained in step (6d) with a diluent to obtain cell-free fat extract D.

In another preferred embodiment, in step (6e), the diluent is water, or PBS solution.

In another preferred embodiment, in step (6e), the mass ratio of the solid content obtained in step (6d) to the diluent is 1:5-1:50, preferably 1:10-1:50, more preferably 1:10-1:30, for example, 1:20.

In another preferred embodiment, in the step (6e), the mixing means stirring, or oscillating to form a uniform lotion.

In another preferred embodiment, step (7) comprises the following steps:

(7a) providing cell-free fat extract D as a form of lotion;
(7b) adding the fat extract C into the lotion obtained in step (7a), and mixing;
(7c) freeze-drying to obtain the decellularized adipose biomaterial.

In another preferred embodiment, in step (7b), the mass ratio of the fat extract C to the lotion obtained in step (7a) is 0.01~100:0.01~100, preferably 0.1~10:0.1~10.

In another preferred embodiment, step (7c) further comprises a step of injecting to a mold first and then freeze-drying.

In another preferred embodiment, in step (7c), the freeze-drying comprises a step of pre-freezing and then freeze-drying.

In another preferred embodiment, in step (7c), the freeze-drying comprises steps of pre-freezing at -50~-150 °C for 12-24 hours, and then freeze-drying for 24-48 hours.

In another preferred embodiment, the decellularized adipose biomaterial has one or more features selected from the group consisting of:

(1) after being filled into the body for 2 weeks, the quality retention rate is above 68%, preferably above 70%, and more preferably above 75%;

(2) after being filled into the body for 2 weeks, the volume retention rate is above 70%, preferably above 72%, more preferably above 75%, and even more preferably above 80%;

(3) after being filled into the body for 3 months, the quality retention rate is above 35%, preferably above 37%, and more preferably above 40%;

(4) after being filled into the body for 3 months, the volume retention rate is above 40%, preferably above 41%, and more preferably above 44%; and

(5) after being stored at 37 °C for 14 days, the protein release of the extracted solution is more than 15 $\mu$g/mL, preferably more than 17 $\mu$g/mL.

In the second aspect of the present invention, provided is a method for preparing the decellularized adipose biomaterial as described in the first aspect of the present invention, comprising the following steps:

(1) providing an adipose tissue raw material, crushing the adipose tissue raw material, and rinsing it to obtain a rinsed adipose tissue;

(2) subjecting the rinsed adipose tissue to centrifugation to obtain a layered mixture, removing the upper oil layer and the lower aqueous layer, and collecting the intermediate layer;

(3) subjecting the intermediate layer to emulsification to obtain an emulsified fat mixture;

(4) subjecting the emulsified fat mixture to centrifugation to obtain an intermediate liquid layer, which is the initial adipose extract A, and an intermediate solid layer, which is the initial adipose extract B;

(5) subjecting the initial adipose extract A to filtration and sterilization to obtain cell-free fat extract C;

(6) subjecting the initial adipose extract B to emulsification treatment, disinfection treatment, decellularization treatment, and delipidation treatment in sequence to obtain cell-free fat extract D;

(7) mixing the cell-free fat extract C with D and post-processing to obtain the decellularized adipose biomaterial.

In another preferred embodiment, the method is as described in the first aspect of the present invention.

In the third aspect of the present invention, provided is a medical material comprising the decellularized adipose biomaterial as described in the first aspect of the present invention, and pharmaceutically acceptable carriers.

In the fourth aspect of the present invention, provided is a medical aesthetic material comprising the decellularized adipose biomaterial as described in the first aspect of the present invention, and acceptable carriers in the field of medical cosmetology.

In the fifth aspect of the present invention, provided is a tissue repair material comprising the decellularized adipose biomaterial as described in the first aspect of the present invention.

In another preferred embodiment, the tissue repair material is an injectable tissue repair material.

In another preferred embodiment, the tissue repair material further comprises acceptable carriers.

In another preferred embodiment, the tissue repair material further comprises a flowing agent selected from the group consisting of physiological saline, PBS buffer, glycerol, polyethylene glycol, and combinations thereof.

In another preferred embodiment, the tissue repair material is applied to the site in need in need of tissue repair through injection filling or direct filling of a surgical opening.

In the sixth aspect of the present invention, provided is a use of the decellularized adipose biomaterial as described in the first aspect of the present invention for preparing a medical material or a medical aesthetic material, wherein the medical material is used for soft tissue repair, and the medical aesthetic material is used for plastic surgery and filling.

In another preferred embodiment, the soft tissue repair comprises one or more features selected from the following group:

(1) promoting fibroblast proliferation;

(2) promoting collagen fiber proliferation; and/or

(3) promoting adipocyte regeneration.

In another preferred embodiment, the plastic surgery and filling comprises one or more features selected from the following group:

(1) promoting fibroblast proliferation;

(2) promoting collagen fiber proliferation; and/or

(3) promoting adipocyte regeneration.

In the seventh aspect of the present invention, provided is a method for soft tissue repair, comprising administering the decellularized adipose biomaterial as described in the first aspect of the present invention to a subject in need thereof.

In another preferred embodiment, the decellularized adipose biomaterial is administered by injection, orally, or topically.

In another preferred embodiment, the injection includes subcutaneous injection, intradermal injection, or intravenous injection.

In the eighth aspect of the present invention, provided is a method for plastic surgery and filling, comprising administering the decellularized adipose biomaterial as described in the first aspect of the present invention to a subject in need thereof.

In another preferred embodiment, the decellularized adipose biomaterial is administered by injection, orally, or topically.

In another preferred embodiment, the injection includes subcutaneous injection, intradermal injection, and intravenous injection.

It should be understood that within the scope of the present invention, the above-described technical features of the present invention and the technical features described in detail below (e.g., embodiments) may be combined with each other to constitute a new or preferred technical solution. Limited by space, it will not be repeated here.

## DETAILED DESCRIPTION OF THE INVENTION

After extensive and in-depth research, the inventors have discovered for the first time a cell-free decellularized adipose biomaterial rich in a mixture of adipose active proteins. This biomaterial retains the active protein factors of human tissue itself, which is more active and has higher biological safety compared to recombinant active protein factors. The active protein is evenly dispersed in the biomaterial, which can achieve sustained release of the active protein in the damaged tissue area, and has a very long-lasting repair effect, thereby accurately promoting angiogenesis at the wound site for a long time. In addition, freeze-dried biomaterial has porous structure that promotes the rapid repair of the tissues and can be stored for a long time. On this basis, the present invention has been completed.

## DESCRIPTION OF THE DRAWINGS

Figure 1 shows the solid content obtained in step (b4) of Example 1 of the present invention. (The left side is treated with ether, and the right side is treated with isopropanol)

Figure 2 is a histological evaluation chart of the materials in animal experiments of the present invention, showing the degradation and cell proliferation promotion results in Examples 1 and 2, as well as Comparative Examples 2 and 3.

### Terms

Unless otherwise defined, all technical and scientific terms used herein have the same meanings as commonly understood by those of ordinary skill in the art to which the present invention belongs.

As used herein, the terms "comprise", "include", and "contain" are used interchangeably to include not only closed definitions, but also semi-closed, and open definitions. In other words, the terms include "consist of" and "substantially consist of".

As used herein, when referring to specific listed values, the term "about" means that the value can vary by no more than 1% from the listed values. For example, as used herein, the expression "about 100" includes all values between 99 and 101 (e.g. 99.1, 99.2, 99.3, 99.4, etc.).

In the present invention, the term "room temperature" refers to 10-40 °C.

**Decellularized adipose biomaterial**

As used herein, the terms "decellularized adipose biomaterial" and "biomaterial of the present invention" can be used interchangeably, referring to the decellularized adipose biomaterial prepared by mixing a liquid cell-free fat extract of a fat mixture acquired by washing, crushing and separating an adipose tissue, with a solid cell-free fat extract acquired from a fat mixture by decellularization, delipidation and other processes.

The biomaterial of the present invention includes a cell-free fat extract without added components (i.e., cell-free fat extract C, CEFFE) and a decellularized fat matrix (i.e., cell-free fat extract D).

The cell-free fat extract refers to the extract (or extracting solution) obtained by filtering and sterilizing the intermediate cell-free liquid, which is obtained by separating the emulsified fat and then removing residual oil, adipose tissue, and cell/tissue debris precipitates. The cell-free fat extract contains various growth factors, such as insulin-like growth factor 1, brain-derived neurotrophic factor, glial cell line-derived neurotrophic factor, basic fibroblast growth factor, vascular endothelial growth factor, transforming growth factor-β, hepatocyte growth factor, and platelet-derived growth factor.

The decellularized fat matrix refers to the extract obtained by further emulsifying, disinfecting, decellularizing, and defatting the cell/tissue debris precipitate separated from emulsified fat. The decellularized fat matrix contains substances such as macromolecular collagen, glycosaminoglycans, elastin, laminin, fibronectin, etc. These substances themselves are excellent tissue repair materials and can be used as sustained-release carriers for CEFFE.

The biomaterial of the present invention is a solid block material, and the active protein is uniformly dispersed in the matrix or carrier of the biomaterial through the process, with excellent sustained release effect. It can be applied by injection to the site in need of tissue repair and/or plastic surgery and filling, with long-term repair effect.

The biomaterial of the present invention is a fat extract without added components, which is a fat extract derived from adipose tissue without adding any solution, solvent, small molecule, chemical agent, or biological additive during the preparation process (except for the rinsing step).

A typical method for preparing the biomaterial of the present invention is as described in the second aspect of the present invention. Furthermore, it should be understood that although the biomaterial of the present invention does not require the addition of any additives (or ingredients) during the preparation process, some or a small amount of safe substances that do not have a negative or adverse effect on the activity of the extract of the present invention can also be added (such as a small amount of water, PBS buffer solution, physiological saline, etc.).

**Preparation method of decellularized adipose biomaterial**

The present invention provides a method for preparing decellularized adipose biomaterial, comprising the following steps:

(1) providing an adipose tissue raw material, crushing the adipose tissue raw material, and rinsing it (such as with physiological saline) to obtain a rinsed adipose tissue;
(2) subjecting the rinsed adipose tissue to centrifugation to obtain a layered mixture, removing the upper oil layer and the lower aqueous layer, and collecting the intermediate layer;
(3) subjecting the intermediate layer to emulsification to obtain an emulsified fat mixture;
(4) subjecting the emulsified fat mixture to centrifugation to obtain an intermediate liquid layer, which is the initial adipose extract A, and an intermediate solid layer, which is the initial adipose extract B;
(5) subjecting the initial adipose extract A to filtration and sterilization to obtain cell-free fat extract C;
(6) subjecting the initial adipose extract B to emulsification treatment, disinfection treatment, decellularization treatment, and delipidation treatment in sequence to obtain cell-free fat extract D;
(7) mixing the cell-free fat extract C with D, then freeze-drying and sterilizing to obtain the final decellularized adipose biomaterial E.

As preferred technical solutions:
In another preferred embodiment, in step (2), the centrifugation is carried out at 800-2500g, preferably 800-2000g, more preferably 1000-1500g, and most preferably 1100-1300g.

In another preferred embodiment, in step (2), the centrifugation time is 1-15 minutes, preferably 1-10 minutes, more preferably 1-8 minutes, and most preferably 1-5 minutes.

In another preferred embodiment, the centrifugation temperature is 2-6 °C.

In another preferred embodiment, in step (3), the emulsification is mechanical emulsification.

In another preferred embodiment, the mechanical emulsification is carried out by repeatedly pipetting with a syringe (such as blowing 20-200 times, preferably 20-150 times, more preferably 20-100 times, and more preferably 30-50 times).

In another preferred embodiment, the blowing method is that two 10ml injection syringes are connected to a three-way stopcock and repeatedly push-pull mixed at a constant speed.

In another preferred embodiment, in step (3), the emulsification is achieved by crushing with a tissue homogenizer.

In another preferred embodiment, step (4) further comprises freezing and thawing the emulsified fat mixture before centrifuging it.

In another preferred embodiment, after freezing and thawing, the thawed mixture is subjected to centrifugation.

In another preferred embodiment, the freezing temperature is -50 °C to -120 °C, preferably -60 °C to -100 °C, and more preferably -70 °C to -90 °C.

In another preferred embodiment, the thawing temperature is 20-40 °C, preferably 25-40 °C, and more preferably 37 °C.

In another preferred embodiment, the number of freeze-thaw cycles is 1-5 times (preferably 1, 2, 3, or 4 times).

In another preferred embodiment, in step (4), after centrifugation, the emulsified fat mixture is divided into four layers, with the first layer being an oil layer, the second layer being a residual adipose tissue layer, the third layer being a liquid layer (i.e. the intermediate liquid layer), and the fourth layer being a cell/tissue fragment precipitation layer.

In another preferred embodiment, in step (4), the centrifugation is carried out at 800-2500g, preferably 800-2000g, more preferably 1000-1500g, and most preferably 1100-1300g.

In another preferred embodiment, in step (4), the centrifugation time is 1-15 minutes, preferably 1-10 minutes, more preferably 2-8 minutes, and most preferably 3-7 minutes.

In another preferred embodiment, the centrifugation temperature is 2-6 °C.

In another preferred embodiment, in step (4), the first layer, the second layer, the third layer, and the fourth layer are sequentially arranged from top to bottom.

In another preferred embodiment, in step (4), the intermediate liquid layer is a transparent or substantially transparent layer, and the intermediate solid layer is yellow or brown.

In another preferred embodiment, in step (5), the filtration can remove adipocytes from the initial adipose extract.

In another preferred embodiment, in step (5), the filtration and sterilization are performed using a filter (such as a 0.22 $\mu$m microporous membrane).

In another preferred embodiment, the filter is a microporous membrane filter.

In another preferred embodiment, the pore size of the microporous membrane is 0.05-0.8 $\mu$m, preferably 0.1-0.5 $\mu$m, more preferably 0.1-0.4 $\mu$m, more preferably 0.15-0.3 $\mu$m, more preferably 0.2-0.25 $\mu$m, and most preferably 0.22 $\mu$m.

In another preferred embodiment, in step (5), the filtration and sterilization are carried out first through a first filter capable of filtering out cells, and then through a second filter (such as a 0.22 $\mu$m filter) capable of filtering out pathogens (such as bacteria).

In another preferred embodiment, step (5) further comprises aliquoting the fat extract to form aliquoted products. (The aliquoted extract can be stored at -20 °C for later use; can be directly used after thawing at low temperature (such as -4 °C) or at room temperature, or be stored at low temperature (such as 4 °C) for a period of time after thawing before use.)

Furthermore, the preferred method for emulsifying the initial adipose extract B in step (6) is to use a homogenizer for mechanical emulsification and subject to centrifugation to separate the oil layer. The emulsification speed is preferably 5000-15000r/min. The emulsification time is preferably 0.5-5 min. The centrifugation speed is preferably 2000-5000r/min. The centrifugation time is preferably 5-15min.

Furthermore, the solvent used for disinfection treatment is a mixture of peracetic acid and ethanol, the preferred concentration of peracetic acid is 0.1-3% and the preferred concentration of ethanol is 4-24%, the mass ratio of the content to the mixed solution of peracetic acid and ethanol is preferably 1:2-1:20, the treatment time is preferably 1-6 hours, and after treatment, the liquid layer is discarded, and PBS solution and purified water are used to wash in sequence.

Furthermore, SDS, TritonX-100, and sodium deoxycholate are preferred as decellularizing agents, the concentration of decellularizing agent is preferably 0.5-5%, the mass ratio of the content to the decellularizing agent solution is preferably 1:2-1:20, the treatment time is preferably 12h-72 hours, and after treatment, the liquid layer is discarded, and PBS solution and purified water are used to wash in sequence.

Furthermore, isopropanol, ethanol, and ether are preferred as degreasers, the mass ratio of the content to the decellularizing agent solution is preferably 1:2-1:10, the treatment time is preferably 2-8 hours, and after treatment, the liquid layer is discarded, and PBS solution and purified water are used to wash in sequence.

Furthermore, the effectiveness of impurity removal can be adjusted by controlling either the mass ratio of the solid content to the reagents in each step, or the processing time.

Furthermore, the method of mixing cell-free fat extract C and D in step (7) is as follows:

(1) adding a certain amount of fat extract D into water, and stirring evenly to form a uniform lotion;

(2) slowly adding the fat extract C into the above uniform lotion and mixing well;

(3) injection molding and freeze-drying to obtain the decellularized adipose biomaterial E.

Furthermore, the freeze-drying process condition is pre-freezing for 1-24 hours and freeze-drying for 24-72 hours.

**Tissue repair material**

The decellularized adipose biomaterial of the present invention prepared by freeze-drying can preserve the active protein factors in the material for a long time without deactivation.

In practical applications, the decellularized adipose biomaterial described in the present invention can be mixed with physiological saline, PBS buffer, glycerol, polyethylene glycol and other solutions in a certain mass ratio. Mixing with these materials for use can endow the decellularized adipose biomaterial described in the present invention with corresponding fluidity for injection into irregular tissue defect sites, facilitating clinical use.

Therefore, another aspect of the present invention also includes a composition of decellularized adipose biomaterial, wherein the decellularized adipose biomaterial described in the present invention can be mixed with carriers to form a flowable injectable tissue repair material.

**Medical material and administration mode**

Due to the excellent activity of promoting tissue repair and enhancing fibrin regeneration of the biomaterial of the present invention, the biomaterial of the present invention, as well as tissue repair materials containing the biomaterial of the present invention as the main active ingredient, can be used for soft tissue repair and/or plastic surgery and filling.

The tissue repair material of the present invention comprises a safe and effective amount of the biological material of the present invention and pharmaceutically acceptable excipients or carriers. The term "safe and effective amount" refers to an amount of the material that is sufficient to significantly improve the condition without causing serious side effects. The effective therapeutic amount is determined based on the age, condition, and course of treatment of the treatment subject. Typically, a tissue repair material contains 1-2000 mg of the biomaterial of the present invention per dose, and more preferably 10-1000 mg of the biomaterial of the present invention per dose.

The "pharmaceutically acceptable carrier" refers to one or more compatible solid or liquid fillers or gel substances, which are suitable for human use and must have sufficient purity and sufficient low toxicity. "Compatibility" herein refers to the ability of each component in the composition to blend with the biomaterial of the present invention and with each other without significantly reducing the efficacy of the biomaterial. Some examples of pharmaceutically acceptable carrier include sugar (such as glucose, sucrose, lactose, etc.), gelatin, vegetable oils (such as soybean oil, sesame oil, peanut oil, olive oil, etc.), polyols (such as propylene glycol, glycerol, mannitol, sorbitol, etc.), emulsifiers (such as Tween), wetting agents (such as sodium dodecyl sulfate), colorants, seasonings, stabilizers, antioxidants, preservatives, pyrogen free water and the like.

The administration method of the biomaterial or medical or medical aesthetic material of the present invention is not particularly limited, and representative administration methods include (but are not limited to): parenteral (intravenous, intramuscular or subcutaneous) administration, and topical administration.

Compositions for parenteral injection may include physiologically acceptable sterile aqueous or anhydrous solutions, dispersions, suspensions or lotions, and sterile powders for redissolution into sterile injectable solutions or dispersions. Suitable aqueous and non-aqueous carriers, diluents, solvents, or excipients include water, ethanol, polyols, and suitable mixtures thereof.

The dosage form of the biomaterial of the invention for topical administration includes gels, ointments, powders, patches, sprays and inhalers. The active ingredient is mixed with physiologically acceptable carriers and any preservatives or buffering agents under sterile condition.

The biomaterial of the present invention and pharmaceutically acceptable salts thereof can be administered separately or in combination with other pharmaceutically acceptable therapeutic agents.

The treatment method of the present invention can be used alone or in combination with other treatment methods or drugs.

When using a pharmaceutical composition, a safe and effective amount of the biomaterial of the present invention is administered to a mammal in need of treatment (such as a human), wherein the dosage at the time of administration is the pharmaceutically effective dosage. For a person weighing 60 kg, the daily dosage is usually 1-2000 mg, preferably 50-1000 mg. Of course, the specific dosage should also consider factors such as the route of administration and the patient's health condition, which are within the skill range of skilled physicians.

**The main advantages of the present invention include:**

(1) In the process of fat decellularization, the present invention first extracts a mixture rich in fat active proteins through an additive-free method, ensuring that it is cell-free, sterile, and virus-free, thereby avoiding the removal and contamination of these active protein factors by chemical or biological reagents during decellularization process. The obtained decellularized adipose biomaterial retains a large amount of active protein factors compared to known processes.

(2) The decellularized adipose biomaterial of the present invention retains the active protein factors of human tissue itself, and has stronger activity and higher biological safety compared to recombinant active protein factors.

(3) The active proteins in the decellularized adipose biomaterial of the present invention are uniformly dispersed in the biomaterial by the process and adhere to the collagen fibers in the biomaterial. During use, the active proteins can be sustained-released in the damaged tissue area (can remain effective for 3 to 6 months), which can accurately promote angiogenesis at the wound site for a long time, thereby achieving the goal of repairing tissue function.

(4) The decellularized adipose biomaterial after freeze-drying has a porous structure, which can be stored for a long time and is easy to achieve industrial production.

(5) The decellularized adipose biomaterial of the present invention does not introduce exogenous proteins or materials and has good biocompatibility with human tissues.

The present invention will be further explained below in combination with specific examples. It should be understood that these examples are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

**Example 1**

The present invention prepares a decellularized adipose biomaterial according to the following method, which includes the steps of preparing initial adipose extract A and initial adipose extract B, as well as the steps of preparing decellularized adipose biomaterial.

a. Preparation of initial adipose extract A and initial adipose extract B

(a1) Adipose tissue was obtained from 6 healthy women who underwent routine liposuction, with an average age of 31 years (24-36 years). After local anesthesia with tumescent fluid injection, a 3mm liposuction cannula with a large side hole (2mm $\times$ 7mm) connecting a 20mL syringe was used to perform radial suction under artificial negative pressure. The obtained fat was allowed to stand upright and stationary. After removing the tumescent fluid, it was rinsed three times with physiological saline.

(a2) The rinsed adipose tissue was taken and placed in a centrifuge tube, and centrifuged at 1200g at 4 °C for 3 minutes in a centrifuge to obtain a layered mixture.

(a3) The upper oil layer and the lower aqueous layer were removed from the layered mixture, and the intermediate layer (i.e., the fat layer containing adipocytes) was collected.

(a4) For the intermediate layer, two 10ml injection syringes were connected to a three-way stopcock and repeatedly push-pull mixed at a constant speed for 30 times to perform mechanical emulsification, and a mechanically emulsified fat mixture (also known as nanofat) was obtained.

(a5) The mechanically emulsified fat mixture was placed in a -80 °C freezer, then thawed in a 37 °C water bath. After a single freeze-thaw cycle, the thawed fat mixture was centrifuged at 1200g at 4 °C for 5 minutes to obtain a layered mixture. The layered mixture was divided into 4 layers: the first layer was an oil layer, the second layer was a residual adipose tissue layer, the third layer was a liquid layer, and the fourth layer was a cell/tissue fragment precipitation layer.

(a6) The oil layer and the cell/tissue fragment precipitation layer were removed, the liquid layer was taken as the initial adipose extract A, and the residual adipose tissue layer (solid layer) was taken as the initial adipose extract B.

(a7) The obtained initial adipose extract A was filtered through a 0.22 $\mu$m filter to sterilize and remove any live cells that may be mixed, thereby obtaining cell-free fat extract C (CEFFE), which was aliquoted and frozen at -20 °C, and thawed at 4 °C for use.

b. Preparation of decellularized adipose biomaterial

(b1) The initial adipose extract B was emulsified at 10000r/min for 2 minutes using a homogenizer, centrifuged at 4000r/min for 10 minutes to separate the oil layer and obtain a solid content.

(b2) The solid content obtained in step (b1) was added at a mass ratio of 1:10 to a peracetic acid/ethanol (with a concentration of 0.5%/4%) disinfectant and oscillated for 4 hours. The liquid layer was discarded after treatment, and the solid content was oscillated and washed with PBS solution and purified water in sequence to obtain a solid content.

(b3) The solid content obtained in step (b2) was added at a mass ratio of 1:10 to TritonX-100 (with a concentration of 1%) and oscillated for 72 hours. The solution was changed every 8 hours. The liquid layer was discarded after treatment, and the solid content was oscillated and washed with PBS solution and purified water in sequence to obtain a solid content.

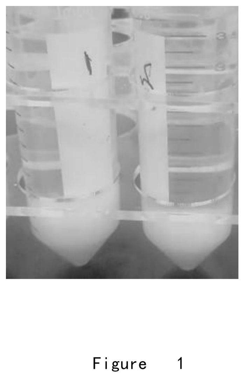

(b4) The solid content obtained in step (b3) was added to ether at a mass ratio of 1:6 and oscillated for 3 hours, or to isopropanol at a mass ratio of 1:10 and oscillated for 3 hours. The liquid layer was discarded after treatment, and the solid content was oscillated and washed with PBS solution and purified water in sequence to obtain a solid content. The solid contents obtained using isopropanol and ether were shown in Figure 1.

(b5) The solid content obtained in step (b4) was added to 20 times of the mass of purified water, the mixture was stirred evenly to form a uniform lotion (cell-free fat extract D), and the fat extract C solution was slowly added to the above uniform lotion and mixed evenly. The mixture was injected into the mold, pre-frozen at -80 °C for 12 hours, and freeze-dried for 48 hours to obtain decellularized adipose biomaterial.

**Example 2**

The present invention prepares a decellularized adipose biomaterial according to the following method, which includes the steps of preparing initial adipose extract A and initial adipose extract B, as well as the steps of preparing decellularized adipose biomaterial. The difference between Example 2 and Example 1 was that in step (a4), the intermediate layer was emulsified using a homogenizer at 12000 r/min for 3 minutes to obtain a mechanically emulsified fat mixture.

a. Preparation of initial adipose extract A and initial adipose extract B

(a1) Adipose tissue was obtained from 6 healthy women who underwent routine liposuction, with an average age of 31 years (24-36 years). After local anesthesia with tumescent fluid injection, a 3mm liposuction cannula with a large side hole (2mm × 7mm) connecting a 20mL syringe was used to perform radial suction under artificial negative pressure. The obtained fat was allowed to stand upright and stationary. After removing the tumescent fluid, it was rinsed three times with physiological saline.

(a2) The rinsed adipose tissue was taken and placed in a centrifuge tube, and centrifuged at 1200g at 4 °C for 3 minutes in a centrifuge to obtain a layered mixture.

(a3) The upper oil layer and the lower aqueous layer were removed from the layered mixture, and the intermediate layer (i.e., the fat layer containing adipocytes) was collected.

(a4) The intermediate layer was emulsified with a homogenizer at 12000r/min for 3 minutes to obtain a mechanically emulsified fat mixture.

(a5) The mechanically emulsified fat mixture was placed in a -80 °C freezer, then thawed in a 37 °C water bath. After a single freeze-thaw cycle, the thawed fat mixture was centrifuged at 1200g at 4 °C for 5 minutes to obtain a layered mixture. The layered mixture was divided into 4 layers: the first layer was an oil layer, the second layer was a residual adipose tissue layer, the third layer was a liquid layer, and the fourth layer was a cell/tissue fragment precipitation layer.

(a6) The oil layer and the cell/tissue fragment precipitation layer were removed, the liquid layer was taken as the initial adipose extract A, and the residual adipose tissue layer (solid layer) was taken as the initial adipose extract B.

(a7) The obtained initial adipose extract A was filtered through a 0.22 μm filter to sterilize and remove any live cells that may be mixed, thereby obtaining cell-free fat extract C (CEFFE), which was aliquoted and frozen at -20 °C, and thawed at 4 °C for use.

b. Preparation of decellularized adipose biomaterial

(b1) The initial adipose extract B was emulsified at 10000r/min for 2 minutes using a homogenizer, centrifuged at 4000r/min for 10 minutes to separate the oil layer and obtain a solid content.

(b2) The solid content obtained in step (b1) was added at a mass ratio of 1:10 to a peracetic acid/ethanol (with a concentration of 0.5%/4%) disinfectant and oscillated for 4 hours. The liquid layer was discarded after treatment, and the solid content was oscillated and washed with PBS solution and purified water in sequence to obtain a solid content.

(b3) The solid content obtained in step (b2) was added at a mass ratio of 1:10 to TritonX-100 (with a concentration of 1%) and oscillated for 72 hours. The solution was changed every 8 hours. The liquid layer was discarded after treatment, and the solid content was oscillated and washed with PBS solution and purified water in sequence to obtain a solid content.

(b4) The solid content obtained in step (b3) was added to ether at a mass ratio of 1:6 and oscillated for 3 hours. The liquid layer was discarded after treatment, and the solid content was oscillated and washed with PBS solution and purified water in sequence to obtain a solid content.

(b5) The solid content obtained in step (b4) was added to 20 times of the mass of purified water, the mixture was stirred evenly to form a uniform lotion (cell-free fat extract D), and the fat extract C solution was slowly added to the above uniform lotion and mixed evenly. The mixture was injected into the mold, pre-frozen at -80 °C for 12 hours, and freeze-dried for 48 hours to obtain decellularized adipose biomaterial.

## Comparative Example 1

The difference between Comparative Example 1 and Example 1 is that only cell-free fat extract C was prepared, which included the steps of preparing initial adipose extract A and preparing cell-free fat extract C.

a. Preparation of initial adipose extract A and cell-free fat extract C

(a1) Adipose tissue was obtained from 6 healthy women who underwent routine liposuction, with an average age of 31 years (24-36 years). After local anesthesia with tumescent fluid injection, a 3mm liposuction cannula with a large side hole (2mm × 7mm) connecting a 20mL syringe was used to perform radial suction under artificial negative pressure. The obtained fat was allowed to stand upright and stationary. After removing the tumescent fluid, it was rinsed three times with physiological saline.

(a2) The washed adipose tissue was taken and placed in a centrifuge tube. It was centrifuged at 1200g at 4 °C for 3 minutes in a centrifuge to obtain a layered mixture.

(a3) The upper oil layer and lower aqueous layer was removed from the layered mixture, and the middle layer (i.e., the fat layer containing adipocytes) was collected.

(a4) For the intermediate layer, two 10ml injection syringes are connected to a tee tube and repeatedly push-pull mixed at a constant speed for 30 times to perform mechanical emulsification, and a mechanically emulsified fat mixture was obtained.

(a5) The mechanically emulsified fat mixture was placed in a -80 °C freezer, then thawed in a 37 °C water bath. After a single freeze-thaw cycle, the thawed fat mixture was centrifuged at 1200g at 4 °C for 5 minutes to obtain a layered mixture. The layered mixture was divided into 4 layers: the first layer was an oil layer, the second layer was a residual adipose tissue layer, the third layer was a liquid layer, and the fourth layer was a cell/tissue fragment precipitation layer.

(a6) The oil layer and the cell/tissue fragment precipitation layer were removed, and the liquid layer was taken as the initial adipose extract A.

(a7) The obtained initial adipose extract A was filtered through a 0.22 $\mu$m filter to sterilize and remove any live cells that may be mixed, thereby obtaining cell-free fat extract C (CEFFE), which was injected into the mold, pre-frozen at -80 °C for 12 hours, and freeze-dried for 48 hours to obtain decellularized adipose biomaterial.

## Comparative Example 2

Comparative Example 2 prepared a decellularized adipose biomaterial according to the following method, which differs from Example 1 in that cell-free fat extract C was not prepared. The method included the step of preparing the initial adipose extract B, as well as the step of preparing decellularized adipose biomaterial.

a. Preparation of initial adipose extract B

(a1) Adipose tissue was obtained from 6 healthy women who underwent routine liposuction, with an average age of 31 years (24-36 years). After local anesthesia with tumescent fluid injection, a 3mm liposuction cannula with a large side hole (2mm × 7mm) connecting a 20mL syringe was used to perform radial suction under artificial negative pressure. The obtained fat was allowed to stand upright and stationary. After removing the tumescent fluid, it was rinsed three times with physiological saline.

(a2) The rinsed adipose tissue was taken and placed in a centrifuge tube, and centrifuged at 1200g at 4 °C for 3 minutes in a centrifuge to obtain a layered mixture.

(a3) The upper oil layer and the lower aqueous layer were removed from the layered mixture, and the intermediate layer (i.e., the fat layer containing adipocytes) was collected.

(a4) For the intermediate layer, two 10ml injection syringes are connected to a three-way stopcock and repeatedly push-pull mixed at a constant speed for 30 times to perform mechanical emulsification, and a mechanically emulsified fat mixture was obtained.

(a5) The mechanically emulsified fat mixture was placed in a -80 °C freezer, then thawed in a 37 °C water bath. After a single freeze-thaw cycle, the thawed fat mixture was centrifuged at 1200g at 4 °C for 5 minutes to obtain a layered mixture. The layered mixture was divided into 4 layers: the first layer was an oil layer, the second layer was a residual adipose tissue layer, the third layer was a liquid layer, and the fourth layer was a cell/tissue fragment precipitation layer.

(a6) The oil layer and the cell/tissue fragment precipitation layer were removed, the residual adipose tissue layer (solid layer) was taken as the initial adipose extract B.

b. Preparation of decellularized adipose biomaterial

(b1) The initial adipose extract B was emulsified at 10000r/min for 2 minutes using a homogenizer, centrifuged at 4000r/min for 10 minutes to separate the oil layer and obtain a solid content.

(b2) The solid content obtained in step (b1) was added at a mass ratio of 1:10 to a peracetic acid/ethanol (with a concentration of 0.5%/4%) disinfectant and oscillated for 4 hours. The liquid layer was discarded after treatment, and the solid content was oscillated and washed with PBS solution and purified water in sequence to obtain a solid content.

(b3) The solid content obtained in step (b2) was added at a mass ratio of 1:10 to TritonX-100 (with a concentration of 1%) and oscillated for 72 hours. The solution was changed every 8 hours. The liquid layer was discarded after treatment, and the solid content was oscillated and washed with PBS solution and purified water in sequence to obtain a solid content.

(b4) The solid content obtained in step (b3) was added to ether at a mass ratio of 1:6 and oscillated for 3 hours. The liquid layer was discarded after treatment, and the solid content was washed with PBS solution and purified water in sequence to obtain a solid content.

(b5) The solid content obtained in step (b4) was added to 20 times of the mass of purified water, the mixture was stirred evenly to form a uniform lotion (cell-free fat extract D), which was injected into the mold, pre-frozen at -80 °C for 12 hours, and freeze-dried for 48 hours to obtain decellularized adipose biomaterial.

**Comparative Example 3**

Comparative Example 3 prepared a decellularized adipose biomaterial according to the following method. The difference from Example 1 was that cell-free fat extract C was not prepared, but vascular endothelial growth factor (VEGF) was added in step b. when preparing the decellularized adipose biomaterial. This method included the steps of preparing the initial adipose extract B and adding vascular endothelial growth factor (VEGF) to prepare the decellularized adipose biomaterial.

a. Preparation of initial adipose extract B

(a1) Adipose tissue was obtained from 6 healthy women who underwent routine liposuction, with an average age of 31 years (24-36 years). After local anesthesia with tumescent fluid injection, a 3mm liposuction cannula with a large side hole (2mm × 7mm) connecting a 20mL syringe was used to perform radial suction under artificial negative pressure. The obtained fat was allowed to stand upright and stationary. After removing the tumescent fluid, it was rinsed three times with physiological saline.

(a2) The rinsed adipose tissue was taken and placed in a centrifuge tube, and centrifuged at 1200g at 4 °C for 3 minutes in a centrifuge to obtain a layered mixture.

(a3) The upper oil layer and the lower aqueous layer were removed from the layered mixture, and the intermediate layer (i.e., the fat layer containing adipocytes) was collected.

(a4) For the intermediate layer, two 10ml injection syringes are connected to a three-way stopcock and repeatedly push-pull mixed at a constant speed for 30 times to perform mechanical emulsification, and a mechanically emulsified fat mixture was obtained.

(a5) The mechanically emulsified fat mixture was placed in a -80 °C freezer, then thawed in a 37 °C water bath. After a single freeze-thaw cycle, the thawed fat mixture was centrifuged at 1200g at 4 °C for 5 minutes to obtain a layered mixture. The layered mixture was divided into 4 layers: the first layer was an oil layer, the second layer was a residual adipose tissue layer, the third layer was a liquid layer, and the fourth layer was a cell/tissue fragment precipitation layer.

(a6) The oil layer and the cell/tissue fragment precipitation layer were removed, and the residual adipose tissue layer (solid layer) was taken as the initial adipose extract B.

b. Preparation of decellularized adipose biomaterial

(b1) The initial adipose extract B was emulsified at 10000r/min for 2 minutes using a homogenizer, centrifuged at 4000r/min for 10 minutes to separate the oil layer and obtain a solid content.

(b2) The solid content obtained in step (b1) was added at a mass ratio of 1:10 to a peracetic acid/ethanol (with a concentration of 0.5%/4%) disinfectant and oscillated for 4 hours. The liquid layer was discarded after treatment, and the solid content was oscillated and washed with PBS solution and purified water in sequence to obtain a solid content.

(b3) The solid content obtained in step (b2) was added at a mass ratio of 1:10 to TritonX-100 (with a concentration of 1%) and oscillated for 72 hours. The solution was changed every 8 hours. The liquid layer was discarded after treatment, and the solid content was oscillated and washed with PBS solution and purified water in sequence to obtain a solid content.

(b4) The solid content obtained in step (b3) was added to ether at a mass ratio of 1:6 and oscillated for 3 hours. The liquid layer was discarded after treatment, and the solid content was washed with PBS solution and purified water in sequence to obtain a solid content.

(b5) The solid content obtained in step (b4) was added to 20 times of the mass of purified water, the mixture was stirred evenly to form a uniform lotion (cell-free fat extract D), and VEGF solution was slowly added to the above uniform lotion and mixed evenly. The mixture was injected into the mold, pre-frozen at -80 °C for 12 hours, and freeze-dried for 48 hours to obtain decellularized adipose biomaterial.

**Test Example 1**

The materials of Example 1 and Comparative Examples 1-3 were subjected to *in vitro* degradation tests according to the method in GB/T 16886.13 to determine the protein content in the extracted solution to reflect the protein release of the materials. The specific operation was as follows:

The sample was dried in a 25 °C vacuum oven to a constant weight, 20mg of the sample was weighed and placed in a 50ml polypropylene centrifuge tube. 10ml PBS buffer solution was added. The centrifuge tube was covered tightly, placed in a 37 °C constant temperature incubator for 14 days, and then centrifuged at 7000r/min for 5 minutes. The supernatant was taken and the protein content of the extracted solution was measured using the BCA method.

Protein release of materials in Examples and Comparative Examples were shown in Table 1. Examples 1, 2 and Comparative Example 3 have higher protein release concentrations, especially Example 2. At the same time, Examples 1 and 2 retain the active proteins of human tissue without introducing exogenous proteins, making them safer compared to Comparative Example 2.

Table 1 protein release of materials

| Case | Protein concentration (ug/ml) |
|---|---|
| Example 1 | 16.31 $\pm$ 1.10 |
| Example 2 | 20.23 $\pm$ 1.78 |
| Comparative Example 1 | 8.58 $\pm$ 1.35 |
| Comparative Example 2 | 7.12 $\pm$ 0.85 |
| Comparative Example 3 | 17.54 $\pm$ 1.34 |

**Test Example 2**

Materials of Examples 1, 2 and Comparative Examples 2, 3 were prepared with physiological saline at a concentration of 6mg/ml. They were then aspirated into syringes and subjected to subcutaneous injection experiments in mice, with 0.2ml of each injection. Samples were collected at 2 weeks, 1 month, and 3 months.

Material retention rate: The collected materials were weighed and placed into a 1ml sealed syringe containing PBS buffer. A 10 μl pipette was used to aspirate the rising solution to the initial mark. The quality retention rate and volume retention rate of materials were calculated and recorded at different sampling points.

Quality retention rate (%) = Quality at the time of material sampling / Initial material quality $\times$ 100%

Volume retention rate (%) = Volume at the time of material sampling / Initial material volume $\times$ 100%

According to the material retention rate results in Tables 2-3, it can be seen that Example 1, Example 2, and Comparative Example 3 have better retention rates in terms of quality and volume compared to Comparative Example 2 at 2 weeks, 1 month, and 3 months after implantation. Based on the results of Test Example 1, we believe that the decellularized adipose biomaterial prepared by the present invention can achieve sustained release of active proteins in subcutaneous tissue area after implantation, promoting tissue regeneration.

Compared to Example 1, Example 2 replaced the method of repeatedly push-pull mixing with a syringe connected to the three-way stopcock at a constant speed with emulsifying with a homogenizer in step (a4). On the one hand, this increases the concentration of adipose active proteins in the final decellularized adipose biomaterial, and at the same time, the structure of the active proteins and biomaterial particles is tighter, resulting in a higher retention rate. This indicates that the present invention can achieve a higher retention rate of decellularized adipose biomaterial by adjusting the process to ensure the long-term effect of promoting tissue regeneration.

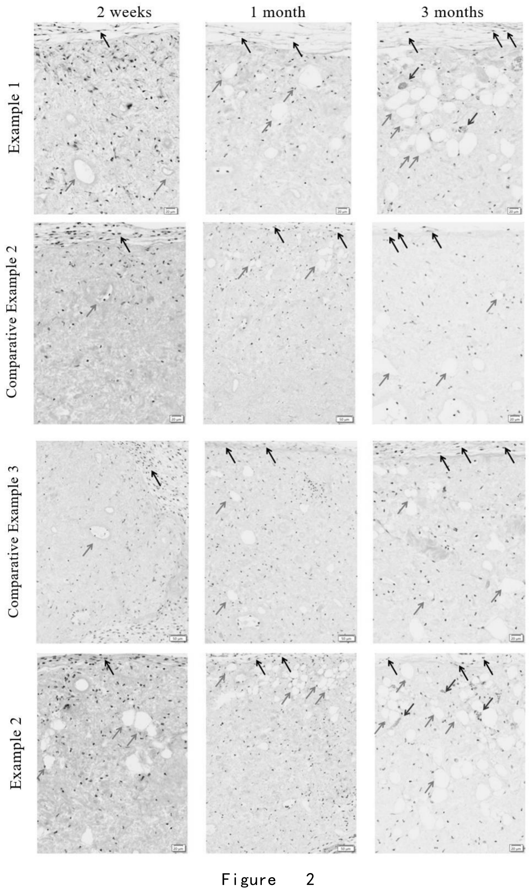

Histological observation: The collected materials were stained with hematoxylin and eosin (H&E). Chromatin in the nucleus and nucleic acids in the cytoplasm appeared purple, and proteins in the cytoplasm and extracellular matrix appeared red or pink, as shown in Figure 2.

At 2 weeks, fibroblast proliferation (black arrow) and fibrosis were observed around each material, with a small amount of infiltration of adipocytes (gray arrow); At 1 month, the material showed certain degradation, with significant fibrosis, proliferation of fibroblasts and collagen fibers around the material, and increased regeneration of adipocytes; At 3 months, the material showed significant degradation and further increase in adipocyte regeneration. The adipocytes in the materials of Examples 1 and 2 was obviously increased and aggregated, accompanied by the generation of new vascular cells (red arrows). However, the number and aggregation degree of adipocytes in Comparative Example 2 were lower than those in Examples 1 and 2. Although VEGF was added in Comparative Example 3, which had a positive effect on the quality and volume retention of the material after implantation, its effect on adipocyte regeneration was weaker than that of the Examples. The decellularized adipose biomaterial prepared by the present invention has excellent effects in promoting fibroblast proliferation, adipose tissue regeneration, and angiogenesis.

Table 2 Quality Retention Rate Data of Materials

| Case | Quality retention rate (%) | | |
|---|---|---|---|
| | 2 weeks | 1 month | 3 months |
| Example 1 | $69.68 \pm 3.34$ | $54.83 \pm 4.08$ | $37.29 \pm 4.61$ |
| Example 2 | $81.64 \pm 9.55$ | $73.13 \pm 5.96$ | $40.27 \pm 0.75$ |
| Comparative Example 2 | $66.45 \pm 2.34$ | $48.39 \pm 11.06$ | $30.04 \pm 1.44$ |
| Comparative Example 3 | $64.75 \pm 3.02$ | $53.65 \pm 4.83$ | $35.43 \pm 2.35$ |

Table 3 Volume Retention Rate Data of Materials

| Case | Volume retention rate (%) | | |
|---|---|---|---|
| | 2 weeks | 1 month | 3 months |
| Example 1 | $72.67 \pm 6.24$ | $61.67 \pm 4.71$ | $41.67 \pm 2.36$ |
| Example 2 | $83.33 \pm 2.36$ | $75.67 \pm 8.50$ | $45.00 \pm 8.16$ |
| Comparative Example 2 | $68.33 \pm 2.36$ | $50.00 \pm 4.08$ | $33.67 \pm 2.36$ |
| Comparative Example 3 | $73.33 \pm 6.24$ | $55.00 \pm 7.07$ | $40.00 \pm 4.08$ |

All documents mentioned herein are incorporated by reference in this application as if each document was individually incorporated by reference. In addition, it should be understood that after reading the above teaching content of the present invention, those skilled in the art can make various changes or modifications to the present invention, and these equivalent forms also fall within the scope defined by the appended claims of the present application.

**Claims**

1. A decellularized adipose biomaterial, wherein, the decellularized adipose biomaterial is prepared by a method comprising the steps of:

(1) providing an adipose tissue raw material, crushing the adipose tissue raw material, and rinsing it to obtain a rinsed adipose tissue;
(2) subjecting the rinsed adipose tissue to centrifugation to obtain a layered mixture, removing the upper oil layer and the lower aqueous layer, and collecting the intermediate layer;
(3) subjecting the intermediate layer to emulsification to obtain an emulsified fat mixture;

(4) subjecting the emulsified fat mixture to centrifugation to obtain an intermediate liquid layer, which is the initial adipose extract A, and an intermediate solid layer, which is the initial adipose extract B;

(5) subjecting the initial adipose extract A to filtration and sterilization to obtain cell-free fat extract C;

(6) subjecting the initial adipose extract B to emulsification treatment, disinfection treatment, decellularization treatment, and delipidation treatment in sequence to obtain cell-free fat extract D;

(7) mixing the cell-free fat extract C with D and post-processing to obtain the decellularized adipose biomaterial.

2. The decellularized adipose biomaterial according to claim 1, wherein in step (3), the emulsification is mechanical emulsification; wherein, the mechanical emulsification is carried out by repeatedly push-pull mixing with a syringe (such as push-pull mixing 20-200 times, preferably 20-150 times, more preferably 20-100 times, and more preferably 30-50 times).

3. The decellularized adipose biomaterial according to claim 1, wherein in step (3), the emulsification comprises breaking and emulsifying by a tissue homogenizer.

4. The decellularized adipose biomaterial according to claim 1, wherein in step (4), after centrifugation, the emulsified fat mixture is layered into 4 layers, with the first layer being an oil layer, the second layer being a residual adipose tissue layer (i.e. the intermediate solid layer), the third layer being a liquid layer (i.e. the intermediate liquid layer), and the fourth layer being a cell/tissue fragment precipitation layer.

5. The decellularized adipose biomaterial according to claim 1, wherein step (6) comprises:

(6a) subjecting the initial adipose extract B to emulsification treatment, and post-processing to obtain a solid content;

(6b) subjecting the solid content obtained in step (6a) to disinfection treatment, and post-processing to obtain a solid content;

(6c) subjecting the solid content obtained in step (6b) to decellularization treatment, and post-processing to obtain a solid content;

(6d) subjecting the solid content obtained in step (6c) to delipidation treatment, and post-processing to obtain a solid content;

(6e) diluting the solid content obtained in step (6d) to obtain cell-free fat extract D.

6. The decellularized adipose biomaterial according to claim 1, wherein in step (6), the emulsification treatment comprises homogenizing and emulsifying with a homogenizer, the emulsification treatment further comprises a step of centrifuging to separate the oil layer, and the emulsification treatment comprises one or more features selected from the group consisting of:

(1) the speed of the homogenizer is 5000-15000 r/min;

(2) the emulsification time is 0.5-15 minutes;

(3) the centrifugation is carried out at 1000-8000 r/min; and

(4) the centrifugation time is 2-20 minutes.

7. The decellularized adipose biomaterial according to claim 1, wherein in step (6), the disinfection treatment comprises mixing with a disinfectant, oscillating and then discarding the liquid layer; and the disinfection treatment further comprises one or more features selected from the group consisting of:

(1) the disinfectant is a mixed solution of peracetic acid and ethanol, the concentration of peracetic acid is 0.05-3%, the concentration of ethanol is 1-24%;

(2) the mass ratio of the solid content obtained after emulsification treatment to the disinfectant is 1:1~1:20; and

(3) the oscillation treatment time is 0.5-6 hours.

8. The decellularized adipose biomaterial according to claim 1, wherein in step (6), the decellularization treatment comprises mixing with a decellularizing agent, oscillating and then discarding the liquid layer; and the decellularization treatment further comprises one or more features selected from the group consisting of:

(1) the decellularizing agent is selected from the group consisting of SDS, TritonX-100, sodium deoxycholate, and combinations thereof;

(2) the concentration of the decellularizing agent is 0.2-5%;

(3) the mass ratio of the solid content obtained after disinfection treatment to the decellularizing agent is 1:1~1:20; and

(4) the oscillation treatment time is 12-96 hours.

9. The decellularized adipose biomaterial according to claim 1, wherein in step (6), the delipidation treatment comprises mixing with a degreaser, oscillating and then discarding the liquid layer; and the delipidation treatment further comprises one or more features selected from the group consisting of:

(1) the degreaser is selected from the group consisting of isopropanol, ethanol, ether, and combinations thereof;
(2) the mass ratio of the solid content obtained after decellularization treatment to the degreaser is 1:1~1:20; and
(3) the oscillation treatment time is 0.5-24 hours.

10. The decellularized adipose biomaterial according to claim 1, wherein step (7) comprises the following steps:

(7a) providing cell-free fat extract D as a form of lotion;
(7b) adding the fat extract C into the lotion obtained in step (7a), and mixing;
(7c) freeze-drying to obtain the decellularized adipose biomaterial.

11. A method for preparing the decellularized adipose biomaterial according to claim 1, comprising the following steps:

(1) providing an adipose tissue raw material, crushing the adipose tissue raw material, and rinsing it to obtain a rinsed adipose tissue;
(2) subjecting the rinsed adipose tissue to centrifugation to obtain a layered mixture, removing the upper oil layer and the lower aqueous layer, and collecting the intermediate layer;
(3) subjecting the intermediate layer to emulsification to obtain an emulsified fat mixture;
(4) subjecting the emulsified fat mixture to centrifugation to obtain an intermediate liquid layer, which is the initial adipose extract A, and an intermediate solid layer, which is the initial adipose extract B;
(5) subjecting the initial adipose extract A to filtration and sterilization to obtain cell-free fat extract C;
(6) subjecting the initial adipose extract B to emulsification treatment, disinfection treatment, decellularization treatment, and delipidation treatment in sequence to obtain cell-free fat extract D;
(7) mixing the cell-free fat extract C with D and post-processing to obtain the decellularized adipose biomaterial.

12. A tissue repair material comprising the decellularized adipose biomaterial according to claim 1.

13. A use of the decellularized adipose biomaterial according to claim 1 for preparing a medical material or a medical aesthetic material, wherein the medical material is used for soft tissue repair, and the medical aesthetic material is used for plastic surgery and filling.

14. A method for soft tissue repair, comprising administering the decellularized adipose biomaterial according to claim 1 to a subject in need thereof.

15. A method for plastic surgery and filling, comprising administering the decellularized adipose biomaterial according to claim 1 to a subject in need thereof.

Figure 1

F i g u r e    2

## INTERNATIONAL SEARCH REPORT

| International application No. |
| --- |
| **PCT/CN2024/090990** |

| A. | CLASSIFICATION OF SUBJECT MATTER |
| --- | --- |
| | A61L 27/36(2006.01)i; A61L 27/50(2006.01)i; A61K35/35(2015.01)i |

According to International Patent Classification (IPC) or to both national classification and IPC

| B. | FIELDS SEARCHED |
| --- | --- |

Minimum documentation searched (classification system followed by classification symbols)

    IPC:A61L,A61K

Documentation searched other than minimum documentation to the extent that such documents are included in the fields searched

Electronic data base consulted during the international search (name of data base and, where practicable, search terms used)

    CNKI, CNABS, DWPI, CNTXT, ENTXTC: CNKI, CNABS, SIPOABS, DWPI, CNTXT, ENTXT: 脂肪, 脱细胞, 破碎, 漂洗, 离心, 中间层, 乳化, 液体层, 初提物, 过滤, 除菌, 无细胞, 消毒, 脱脂, fat, decellulariz+, disrupt+, rins+, centrifug+, intermediate layer, emulsif+, liquid layer, extract+, filter+, steriliz+, cell-free, steriliz+, defat+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 114903920 A (SHANGHAI SEMECELL TECHNOLOGY CO., LTD.) 16 August 2022 (2022-08-16) <br> description, paragraphs [0069]-[0123], and embodiment 1 | 1-15 |
| X | CN 112386528 A (SHANGHAI SEMECELL TECHNOLOGY CO., LTD.) 23 February 2021 (2021-02-23) <br> description, embodiment 1 | 1-15 |
| X | CN 115702907 A (SHANGHAI SEMECELL TECHNOLOGY CO., LTD.) 17 February 2023 (2023-02-17) <br> description, embodiment 1 | 1-15 |
| X | CN 110496241 A (NINTH PEOPLE'S HOSPITAL AFFILIATED TO SHANGHAI JIAO TONG UNIVERSITY SCHOOL OF MEDICINE) 26 November 2019 (2019-11-26) <br> description, embodiment 1 | 1-15 |
| A | CN 108478868 A (YI, Chenggang) 04 September 2018 (2018-09-04) <br> entire document | 1-15 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **04 August 2024** | **09 August 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/090990**

| C. | DOCUMENTS CONSIDERED TO BE RELEVANT | |
|---|---|---|
| Category* | Citation of document, with indication, where appropriate, of the relevant passages | Relevant to claim No. |
| A | WO 2012002986 A2 (THE REGENTS OF THE UNIVERSITY OF CALIFORNIA; CHRISTMAN, KAREN L.; YOUNG, D. ADAM) 05 January 2012 (2012-01-05) entire document | 1-15 |
| A | WO 2017195225 A1 (PROMOITALIA GROUP SPA) 16 November 2017 (2017-11-16) entire document | 1-15 |

Form PCT/ISA/210 (second sheet) (July 2022)

## INTERNATIONAL SEARCH REPORT

| International application No. |
|---|
| **PCT/CN2024/090990** |

---

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **14-15**
   because they relate to subject matter not required to be searched by this Authority, namely:

   Claims 14-15 respectively relate to a soft tissue repair method and a shaping and filling method, which both methods fall within subject matter for which no search is required by the International Searching Authority as listed in PCT Rule 39.1(iv). The subject matter of claim 14 is reasonably expected to be "a method for preparing a soft tissue repair material"; and the subject matter of claim 15 is reasonably expected to be "a method for preparing a shaping and filling material". The search is carried out regarding the amended subject matter which is reasonably expected.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

Form PCT/ISA/210 (continuation of first sheet) (July 2022)

**INTERNATIONAL SEARCH REPORT**
Information on patent family members

International application No.

**PCT/CN2024/090990**

| Patent document cited in search report | | | Publication date (day/month/year) | Patent family member(s) | | | Publication date (day/month/year) |
|---|---|---|---|---|---|---|---|
| CN | 114903920 | A | 16 August 2022 | None | | | |
| CN | 112386528 | A | 23 February 2021 | None | | | |
| CN | 115702907 | A | 17 February 2023 | None | | | |
| CN | 110496241 | A | 26 November 2019 | None | | | |
| CN | 108478868 | A | 04 September 2018 | None | | | |
| WO | 2012002986 | A2 | 05 January 2012 | US | 2012264190 | A1 | 18 October 2012 |
| | | | | WO | 2012002986 | A3 | 08 March 2012 |
| WO | 2017195225 | A1 | 16 November 2017 | None | | | |

Form PCT/ISA/210 (patent family annex) (July 2022)